# EUROPÄISCHE PATENTSCHRIFT

(11) **EP 1 272 140 B1**
(45) Veröffentlichungstag und Bekanntmachung des Hinweises auf die Patenterteilung: **25.04.2007**
(21) Anmeldenummer: 01923651.2
(22) Anmeldetag: 08.03.2001
(51) Int. Cl.: A61F 13/00

(54) **WUNDAUFLAGE MIT VERMINDERTER VERWACHSUNGSTENDENZ**
WOUND DRESSING WITH A REDUCED ACCRETION TENDENCY
PANSEMENT POUR PLAIE A TENDANCE REDUITE A L'ADHERENCE

(30) Priorität: 23.03.2000 DE 10014557
(43) Veröffentlichungstag der Anmeldung: 08.01.2003
(73) Patentinhaber: LTS LOHMANN Therapie-Systeme AG, 56626 Andernach (DE)
(72) Erfinder: BERTHOLD, Achim, 56626 Andernach (DE); MÜLLER, Walter, 56564 Neuwied (DE)
(74) Vertreter: Flaccus, Rolf-Dieter
(86) Internationale Anmeldenummer: PCT/EP2001/002600
(87) Internationale Veröffentlichungsnummer: WO 2001/070153

(56) Entgegenhaltungen:
- EP-A- 0 031 018
- US-A- 5 487 889
- US-A- 5 632 731
- US-A- 5 759 570

## Beschreibung

Die Erfindung betrifft eine Wundauflage zur Förderung der Wundheilung, welche sich dadurch auszeichnet, daß sie keine oder nur eine geringe Verwachsungstendenz mit der Wunde hat.

Wundauflagen müssen eine Reihe verschiedener Anforderungen erfüllen, um die Wundheilung bestmöglich zu unterstützen. Zum einen gilt es, die Wunde vor Umwelteinflüssen, wie z.B. Eindringen von Fremdkörpern und Einwirkung von äußerem Druck, zu schützen. Wundauflagen müssen deshalb keimfrei und sterilisierbar sein und damit die Wahrscheinlichkeit einer Sekundärinfektion senken. Ferner dienen Wundauflagen dazu, die Wunde warm und feucht zu halten, da Wunden besser heilen, wenn sie unter Okklusion feucht gehalten werden. Als Ursache für die "heilende" Wirkung eines okklusiven Milieus wird in der Literatur unter anderem folgender Mechanismus diskutiert: Durch Sauerstoffausschluß ist die Wunde gezwungen, über das Blut Sauerstoff in das Wundgebiet zu bringen. Dies erfolgt durch vermehrte Gefäßeinsprossung unter Gefäßneubildung und damit geförderte Wundheilung.

Andererseits soll die Wundauflage eine gute Saugfähigkeit und eine ausreichende Saugkapazität aufweisen, um ein Abziehen des Wundsekretes zu ermöglichen und die Bildung von feuchten Kammern zu vermeiden. Insbesondere nicht abfließendes, eventuell mit Keimen besetztes Wundsekret birgt ein hohes Infektionsrisiko.

Eine Wundauflage soll außerdem eine ausreichende Naßfestigkeit aufweisen und das Zurückbleiben von Rückständen verhindern, welche die Wundheilung irritieren könnten. Um den Prozeß der Wundheilung zu fördern, sollte eine Wund-Wundauflage eine ausreichende Sauerstoff- und Wasserdampfdurchlässigkeit und damit Atmungsaktivität gewährleisten. Sie soll sich der Wunde anschmiegen, um eine gleichmäßige Aufnahme des Wundsekrets bzw. gegebenenfalls Abgabe von Wirkstoffen zu ermöglichen, hautfreundlich sowie leicht zu applizieren und zu entfernen sein, um die Wundruhe zu gewährleisten.

Im Stand der Technik werden verschiedene Wundauflagen beschrieben. So offenbart US 5,487,889 eine Wundauflage, die ein Reservoir mit sezernierenden Zellen umfaßt, die die Wundheilung fördernde Faktoren absondern. Das Reservoir weist dabei eine permeable Membran auf, die die Zellen zurückhält, nicht aber die sezernierten Faktoren, die durch die Membran in die Wunde gelangen und die Heilung fördern.

In EP 0031018 wird eine Abdeckvorrichtung zur Behandlung von Haut, insbesondere bei Verbrennungen, Wunden oder Hautdefekten, beschrieben, die aus einer auf der Wunde aufliegenden wasserdampf- und gasdurchlässigen Membran, einem auf der Membran liegenden gelartigen Polymerisat und einem mit einer Folie umschlossenen, an die Membran angrenzendem Reservoir eines Wirkstoffs besteht.

In US 5,759,570 wird eine Wundauflage beschrieben, die eine Filtrationsmembran, eine Wundkontaktschicht aus bioabsorbierbaren Material auf der der Haut zugewandten Seite der Membran und eine Absorptionsschicht auf der der Haut abgewandten Seite der Membran enthält. Die Porengröße der Membran ist dabei so gewählt, daß sie Biopolymere mit hohem Molekulargewicht und Wundheilungsfaktoren in der Wunde zurückhält, während das Eindringen von Bakterien verhindert und der schnelle Transport von Exsudat in die Absorptionsschicht ermöglicht wird.

Als nachteilig hat sich bei den bekannten Wundauflagen oft herausgestellt, daß sie dazu neigen, mit der Wundoberfläche zu verkleben. Dies beruht darauf, daß das im Verlauf der Wundheilung neugebildete Gewebe, z. B. Granulationsgewebe, Blutgefäße etc., mit der Unterseite der Wundauflage verwächst, wobei Zellen in die Wundauflage einwandern oder Ausläufer bilden, die in die Wundauflage hineinsprossen. Da bei jedem Verbandswechsel und der damit verbundenen Abnahme des verklebten Verbandes frisches Granulationsgewebe und Epithelsäume mit abgerissen werden, wird auf diese Weise durch derartige Verklebungen der Wundauflage mit der Wunde die Heilung verzögert.

Zwar könnte die Gefahr der Bildung von Verklebungen bzw. Verwachsungen der Wunde mit der Wundauflage dadurch vermindert werden, daß ein häufiger Verbandswechsel vorgenommen wird, so daß eine Verklebung oder Verwachsung aufgrund des kurzen Zeitintervalls nicht entstehen kann. Jedoch ist es vorzuziehen, die Wundauflage nur selten bzw. weniger häufig zu wechseln, um den Heilungsprozeß möglichst wenig zu stören. Da das Abnehmen einer vollgesaugten Wundauflage einen Verlust von Bestandteilen des Wundexsudats und damit immunkompetenter Zellen (das sind Zellen, die Antigene erkennen können und die entsprechenden Antikörper produzieren, und Zellen, die Fremdkörper aufnehmen und somit neutralisieren können) verursacht, wird der Heilungsprozeß bei jedem Wechsel der Wundauflage unterbrochen oder gestört.

Die Aufgabe der vorliegenden Erfindung bestand deshalb darin, eine Wundauflage bereitzustellen, welche nur eine geringe oder keine Tendenz hat, mit der Wunde zu verwachsen. Gleichzeitig soll eine solche Wundauflage den oben erwähnten allgemeinen Anforderungen genügen.

Überraschenderweise wird diese Aufgabe dadurch gelöst, daß bei einer Wundauflage, die eine mindestens einlagige Schicht umfaßt, diese Schicht auf der der Wunde zugewandten und mit der Wunde in Berührung kommenden Seite mit einer porösen Membran oder einer semipermeablen Folie verbunden wird. Diese zwischen der Wundoberfläche und der mindestens einlagigen Schicht der Wundauflage befindliche poröse Membran oder Folie hemmt oder verhindert das Einwachsen oder Hineinsprossen von Zellen in die Folie und in die darüber liegende Schicht. Dadurch werden Verwachsungen der heilenden Wunde mit der Wundauflage unterbunden.

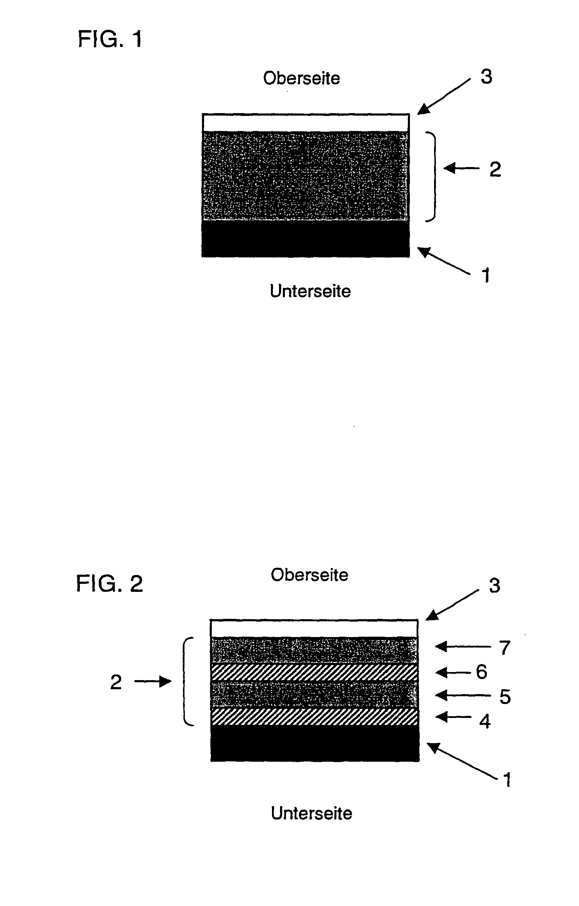

Die erfindungsgemäße Wundauflage wird im folgenden anhand der in Fig. 1 und 2 gezeigten Ausführungsbeispiele näher erläutert. Die Fig. 1 u. 2 zeigen die erfindungsgemäßen Wundauflagen jeweils im Querschnitt, um den Schichtaufbau erkennen zu lassen.

Die erfindungsgemäße Wundauflage mit verminderter Verwachsungstendenz umfaßt in einer einfachen Ausführungsform, wie in Fig. 1 dargestellt, eine poröse Membran oder semipermeable Folie (1) und eine darüberliegende Schicht (2). Die Membran oder Folie (1) bildet die Unterseite der Wundauflage, welche auf die Wundoberfläche aufgelegt wird. Die Schicht (2) bildet die äußere Schicht bzw. Oberseite der Wundauflage.

Als poröse Membranen oder semipermeable Folien eignen sich grundsätzlich Membranen oder Folien, die üblicherweise bei der Mikrofiltration oder Ultrafiltration verwendet werden. Vorzugsweise wird der Porendurchmesser bzw. die Ausschlußgrenze so gewählt, daß es für Gewebszellen nicht mehr möglich ist, in die Membran oder Folie einzuwachsen. Insbesondere eignen sich Membranen, deren Porengröße kleiner als 10 µm, zweckmäßig kleiner als 1 µm und vorzugsweise kleiner als 0,25 µm ist.

Als semipermeable Folien werden solche Folien verstanden, die durchlässig sind für Wasser und darin gelöste Stoffe, einschließlich von Proteinmolekülen. Die einsetzbaren Membranen oder Folien können beispielsweise auf der Basis von Celluloseacetat, Cellulosetriacetat, Cellulosenitrat, Regenerat-Cellulose, Polyvinylalkohol oder Polyamid hergestellt sein.

Die über der Membran oder Folie befindliche und vorzugsweise damit fest verbundene Schicht (2) ist bevorzugt als saugfähige Schicht ausgestaltet, welche der Aufnahme und Ableitung des Wundexsudates dient. Sie kann deshalb saugfähige Materialien enthalten, welche dem Fachmann bekannt sind. Beispielsweise eignen sich hierfür quellfähige Polymere, z. B. Hydrogele auf der Basis von Gelatine, Kollagen, Kollagen-Heparin-Komplexen, Alginaten, Pektinen, Stärken, Albumin, Agarose, Cellulosetypen (z. B. Carboxymethylcellulose, Methylcellulose, Hydroxypropylmethylcellulose), Polyethylenglykolen, Polyvinylpyrrolidonen, Methylpyrrolidon-Chitosan, Cyclo-Dextrinen, Hyaluronsäuren, Polyanhydriden, Polyvinylalkoholen, Polyacrylsäure, Poloxameren und Polyacrylamiden.

Die Außenseite bzw. Oberseite der Wundauflage kann zusätzlich mit einer Deckschicht (3) versehen sein, welche auf der Schicht (2) aufliegt bzw. mit dieser verbunden ist. Sie kann beispielsweise als Schutzschicht dienen, welche der Wundauflage Festigkeit verleiht und diese vor Beschädigungen schützt. Sie kann auch feuchtigkeits- oder wasserdampfundurchlässig sein.

Der Aufbau der erfindungsgemäßen Wundauflage umfaßt außerdem eine wirkstoffundurchlässige Deckschicht sowie gegebenenfalls eine ebenfalls wirkstoffundurchlässige Schutzfolie bzw. Abziehfolie. Als Deckschicht eignen sich vor allem Polyester, darüber hinaus aber nahezu beliebige andere hautverträgliche Kunststoffe, wie Polyvinylchlorid, EthylenVinylacetat-Copolymere, Polyvinylacetat, Polyethylen, Polypropylen, Polyurethane, Cellulosederivate und viele andere mehr. Im Einzelfall kann die Deckschicht mit einer zusätzlichen Auflage versehen werden, z. B. durch Bedampfung mit Metallen, insbesondere Aluminium, oder anderen diffusionssperrenden Zusatzstoffen wie Siliciumdioxid oder ähnlichen, dem Fachmann bekannten Stoffen. Für die ablösbare Schutzschicht können dieselben Materialien verwendet werden wie für die Rückschicht, vorausgesetzt, daß sie durch eine geeignete Oberflächenbehandlung wie Silikonisierung ablösbar ist. Es können aber auch andere ablösbare Schutzschichten wie Polytetrafluorethylen-behandeltes Papier, Cellophan^{®}, Polyvinylchlorid oder ähnliche verwendet werden.

Besonders vorteilhaft sind solche Ausführungsformen der erfindungsgemäßen Wundauflage, bei welchen die Membran oder Folie (1) und die Schicht (2) einen die Wundheilung fördernden pharmazeutischen Wirkstoff, oder mehrere Wirkstoffe, enthalten, der von der oder durch die Membran oder Folie an die Wunde abgegeben werden kann.

Beispielsweise kann die der Wunde zugewandte Außenseite der Membran oder Folie (1) mit Wirkstoff(en) beladen sein und die Schicht (2) kann mit einem oder mehreren Wirkstoff(en) getränkt oder imprägniert sein. Bevorzugt kommen dabei solche Wirkstoffe zum Einsatz, welche die Wundheilung fördern.

Wenn die Wundauflage Wirkstoffe enthält, ist es besonders vorteilhaft, wenn sie gleichzeitig Mittel aufweist, welche eine kontrollierte Freisetzung des bzw. der Wirkstoffe ermöglichen. Dies kann beispielsweise mit Hilfe von die Wirkstoffabgabe steuernden zusätzlichen Membranen erfolgen, oder durch Verwendung von Polymermatrices, welche als Wirkstoffreservoir dienen und aus welchen der Wirkstoff in kontrollierbarer Weise freigesetzt wird.

Als wundheilungsfördernde Wirkstoffe, welche in die erfindungsgemäße Wundauflage inkorporiert werden können, eignen sich vor allem solche, die ausgewählt sind aus der Gruppe der Wachstumsfaktoren, der Antibiotika, der Antiseptika, der Amtimykotika, der Analgetika, der Enzyminhibitoren, der Antihistaminika, der Vitamine, der Glucocorticoide, der antiviralen Wirkstoffe, der Steroide, der Nucleoside einschließlich der Desoxyribonucleoside, der Enzyme (z. B. Proteinkinase C), sowie aus der Gruppe der Hormone.
Aus der Gruppe der Wachstumsfaktoren eignen sich insbesondere Platelet Derived Growth Factor, Epidermal Growth Factor, Platelet Derived Endothelial Cell Growth Factor, Acidic Fibroblast Growth Factor, Basic Fibroblast Growth Factor, Transforming Growth Factor alpha, Transforming Growth Factor beta, Keratinocyte Growth Factor, Insulin-Like Growth Factor 1, Insulin-Like Growth Factor 2 und Tumor Necrosis Factor; von den Wachstumsfaktoren ist der Platelet Derived Growth Factor besonders bevorzugt.

Aus der Gruppe der Hormone werden bevorzugt Insulin, Wachstumshormone, Tyroxin, Trijodthyronin ausgewählt.

Wie aus Fig. 2 ersichtlich ist, schließt die Erfindung auch solche Ausführungsformen mit ein, bei welchen die Schicht (2) aus zwei oder mehreren Lagen aufgebaut ist. Beispielsweise kann diese Schicht aus vier Lagen (4-7) bestehen, von denen z. B. jeweils zwei Lagen (5, 7) polymerhaltig sind und jeweils zwei andere Lagen (4, 6) vlies- oder gewebeartig aufgebaut sind. Ebenso kann die Schicht (2) aus zwei einzelnen Lagen aufgebaut sein, von denen eine auf Polymerbasis hergestellt ist, und die andere aus vlies- oder gewebeartigem Material besteht. Jedoch sind auch eine Vielzahl weiterer Ausführungsformen möglich, die sich durch die Kombination und Anordnung geeigneter Materialien in verschiedenen Lagen der Schicht (2) ergeben können.

Auch die in Fig. 2 gezeigte Ausführungsform weist erfindungsgemäß auf der der Wundseite zugewandten Unterseite eine poröse Membran oder semipermeable Folie (1) auf, um ein Anwachsen der Wundauflage auf der Wunde zu hemmen. Zusätzlich kann eine solche Ausführungsform auf der wundabgewandten Oberseite mit einer Deckschicht (3) ausgestattet sein.

Falls eine Ausführungsform gewählt wird, bei welcher die Schicht (2) zwei- oder mehrlagig aufgebaut ist und Wirkstoff(e) enthält, so ergibt sich zudem die Möglichkeit, daß nur eine oder nur einige dieser Lagen wirkstoffhaltig ausgebildet ist bzw. sind. Auch kann z. B. eine der Lagen als Steuermembran ausgebildet sein, welche die Wirkstoffabgabe aus einer darüber liegenden wirkstoffhaltigen Lage reguliert.

Die erfindungsgemäße Wundauflage mit verminderter Verwachsungstendenz eignet sich zur allgemeinen Verwendung bei der Wundversorgung. Besonders vorteilhaft ist ihre Verwendung zur Behandlung von chronischen oder schlecht heilenden Wunden.

Die erfindungsgemäße Wundauflage wird für die Lagerung zweckmäßig noch auf der Außenseite der Folie bzw. Membran mit einer Abziehfolie versehen, die z. B. aus den für die Deckschicht aufgeführten Materialen bestehen kann, und/oder sie ist in einer Rundumverpackung aus üblichem Material enthalten.

## Patentansprüche

1. Wundauflage, umfassend eine mindestens einlagige Schicht (2), die auf der Wundkontaktseite eine das Hineinwachsen oder Hineinsprossen von Zellen verhindernde poröse Membran oder semipermeable Folie (1) aufweist, **dadurch gekennzeichnet, daß** die Wundauflage mindestens einen die Wundheilung fördernden Stoff umfasst, welcher in der porösen Membran oder semipermeablen Folie (1) enthalten ist, mit dem die der Wunde zugewandte Außenseite der Membran oder Folie (1) beladen ist.

2. Wundauflage nach Anspruch 1, **dadurch gekennzeichnet, daß** auch die Schicht (2) mit einem oder mehreren Wirkstoffen getränkt oder imprägniert sein kann, und der oder die Wirkstoffe in kontrollierter Weise freigesetzt wird/werden.

3. Wundauflage nach Anspruch 1 oder 2, **dadurch gekennzeichnet, daß** die Schicht (2) mindestens teilweise aus saugfähigen Materialien aufgebaut ist oder solche Materialien enthält.

4. Wundauflage nach einem oder mehreren der vorangehenden Ansprüche, **dadurch gekennzeichnet, daß** der Porendurchmesser der porösen Membran so gewählt wird, daß das Eindringen oder Einwachsen von Gewebszellen in die Membran verhindert wird, wobei der Porendurchmesser kleiner als 10 µm, zweckmäßig kleiner als 1 µm und vorzugsweise kleiner als 0,25 µm ist.

5. Wundauflage nach einem oder mehreren der vorangehenden Ansprüche, **dadurch gekennzeichnet, daß** die Schicht (2) auf der außenliegenden, wundabgewandten Seite mit einer Deckschicht (3) versehen ist, welche die äußerste Schicht der Wundauflage bildet.

6. Wundauflage nach einem oder mehreren der vorangehenden Ansprüche, **dadurch gekennzeichnet, daß** die Schicht (2) zwei oder mehrere Lagen umfaßt, wobei vorzugsweise mindestens eine Lage aus vlies- oder gewebeartigem Material aufgebaut ist und mindestens eine weitere Lage aus Polymeren aufgebaut ist.

7. Wundauflage nach einem oder mehreren der vorangehenden Ansprüche, **dadurch gekennzeichnet, daß** die Schicht (2), oder eine die sie bildenden Lagen, und / oder die poröse Membran oder semipermeable Folie (1) mindestens einen die Wundheilung fördernden Wirkstoff enthält.

8. Wundauflage nach einem oder mehreren der vorhergehenden Ansprüche, **dadurch gekennzeichnet, daß** als die Wundheilung fördernder Wirkstoff ein Wachstumsfaktor, insbesondere Platelet Derived Growth Factor, zugegen ist.

9. Wundauflage nach einem oder mehreren der vorhergehenden Ansprüche, **dadurch gekennzeichnet, daß** sie Mittel aufweist, welche eine kontrollierte Freisetzung des/der Wirkstoffe(s) bewirken, wobei es sich bei diesen Mitteln vorzugsweise um eine die Freisetzung steuernde Membran oder eine die Freisetzung steuernde Polymerzusammensetzung handelt.

10. Wundauflage nach einem oder mehreren der vorangehenden Ansprüche, **dadurch gekennzeichnet, daß** sie noch auf der Außenseite der Membran oder Folie mit einer Schutzfolie versehen oder in einer Rundumverpackung enthalten ist.

## Claims

1. Wound dressing, comprising a layer (2) which is at least single-ply and which, on the wound-contact side thereof, has a porous membrane or semi-permeable film (1) which prevents ingrowth or budding of cells into said membrane or film and said layer, **characterised in that** said wound dressing comprises at least one wound healing-promoting substance which is contained in said porous membrane or semipermeable film (1), and that the outer side, facing the wound, of said membrane or film (1) is loaded with said wound healing-promoting substance.

2. Wound dressing according to Claim 1, **characterised in that** also the layer (2) is soaked or impregnated with one ore more active substances, and that the active substance(s) is/are released in a controlled manner.

3. Wound dressing according to Claim 1 or 2, **characterized in that** the layer (2) is at least partially made up of absorptive materials or contains such materials.

4. Wound dressing according to one or more of the preceding claims, **characterized in that** the pore diameter of the porous membrane is selected such that intrusion or ingrowth of tissue cells into the membrane is prevented, the pore diameter being below 10 µm, usefully below 1 µm, and preferably below 0.25 µm.

5. Wound dressing according to one or more of the preceding claims, **characterized in that** the layer (2) is provided with a cover layer (3) on the external side, which is averted from the skin, which cover layer (3) forms the outermost layer of the wound dressing.

6. Wound dressing according to one or more of the preceding claims, **characterized in that** the layer (2) comprises two or more plies, preferably with at least one ply being made up of nonwoven-like material or woven fabric-like material, and at least one further ply being made up of polymers.

7. Wound dressing according to one or more of the preceding claims, **characterized in that** the layer (2), or one of the plies forming said layer, and/or the porous membrane or semipermeable film (1) contains at least one active substance which promotes wound healing.

8. Wound dressing according to one or more of the preceding Claims, **characterized in that** a growth factor, in particular platelet derived growth factor, is added as the active substance promoting wound healing.

9. Wound dressing according to one or more of the preceding Claims, **characterized in that** it comprises means which effect a controlled release of the active substance(s), said means preferably being a release-controlling membrane or a release-controlling polymer composition.

10. Wound dressing according to one or more of the preceding claims, **characterized in that** it is, in addition, provided with a protective film on the outer side of the membrane or film, or that it is contained in a surrounding outer package.

## Revendications

1. Pansement pour plaie, comprenant une couche (2) au moins à une strate, qui présente sur le côté du contact avec la plaie une membrane poreuse ou un film (1) semi-perméable empêchant la croissance ou la poussée de cellules à l'intérieur, **caractérisé en ce que** le pansement pour plaie comprend au moins un tissu favorisant la cicatrisation de la plaie, qui est inclus dans la membrane poreuse ou le film (1) semi-perméable, avec lequel le côté extérieur de la membrane ou du film (1), tourné vers la plaie, est chargé.

2. Pansement pour plaie selon la revendication 1, **caractérisé en ce que** également la couche (2) peut être imbibée ou imprégnée avec un ou plusieurs agents actifs, et le ou les agents actifs est/sont libéré(s) de façon contrôlée.

3. Pansement pour plaie selon la revendication 1 ou 2, **caractérisé en ce que** la couche (2) est conçue au moins partiellement à base de matériaux absorbants ou contient de tels matériaux.

4. Pansement pour plaie selon l'une ou plusieurs des revendications précédentes, **caractérisé en ce que** le diamètre de pore de la membrane poreuse est choisi de telle sorte que la pénétration ou la croissance de cellules de tissu dans la membrane est empêchée, le diamètre de pore étant inférieur à 10 µm, de préférence inférieur à 1 µm et avec une préférence plus grande inférieur à 0,25 µm.

5. Pansement pour plaie selon l'une quelconque ou plusieurs des revendications précédentes, **caractérisé en ce que** la couche (2) est dotée sur le côté situé à l'extérieur et détourné de la plaie d'une couche de recouvrement (3) qui forme la couche extérieure du pansement pour plaie.

6. Pansement pour plaie selon l'une quelconque ou plusieurs des revendications précédentes, **caractérisé en ce que** la couche (2) comprend une ou deux strates, de préférence au moins une strate étant construite à base de matériau non tissé ou de matériau de type tissu et au moins une autre strate étant constituée à base de polymères.

7. Pansement pour plaie selon l'une quelconque ou plusieurs des revendications précédentes, **caractérisé en ce que** la couche (2) ou l'une des strates qui la forme et/ou la membrane poreuse ou le film (1) semi-perméable contient un agent actif favorisant la cicatrisation de la plaie.

8. Pansement pour plaie selon l'une quelconque ou plusieurs des revendications précédentes, **caractérisé en ce qu'**un facteur de croissance, en particulier un Platelet Derived Growth Factor, est présent en tant qu'agent actif favorisant la cicatrisation de la plaie.

9. Pansement pour plaie selon l'une quelconque ou plusieurs des revendications précédentes, **caractérisé en ce qu'**il présente des moyens qui entraînent une libération contrôlée du / des agent(s) actif(s), sachant qu'il s'agit en ce qui concerne ces moyens, de préférence d'une membrane contrôlant la libération ou d'un composé polymère contrôlant la libération.

10. Pansement pour plaie selon l'une quelconque ou plusieurs des revendications précédentes, **caractérisé en ce qu'**il est doté également sur le côté extérieur de la membrane ou du film d'un film de protection ou est inclus dans un emballage complet.
